# EUROPÄISCHE PATENTSCHRIFT

(11) **EP 2 806 927 B1**
(45) Veröffentlichungstag und Bekanntmachung des Hinweises auf die Patenterteilung: **15.03.2017**
(21) Anmeldenummer: 14706768.0
(22) Anmeldetag: 15.01.2014
(51) Int. Cl.: A61M 13/00, A61M 16/08, A61B 1/313

(54) **INSUFFLATIONSSCHLAUCH MIT BEFEUCHTUNGSMATERIAL UND HEIZELEMENT FÜR DIE LAPAROSKOPIE**
INSUFFLATION TUBE COMPRISING A HUMIDIFYING MATERIAL AND A HEATING ELEMENT, FOR LAPAROSCOPY
TUBE D'INSUFFLATION POURVU D'UN AGENT D'HUMIDIFICATION ET D'UN ÉLÉMENT DE CHAUFFE POUR LA LAPAROSCOPIE

(30) Priorität: 15.01.2013 DE 102013000492; 18.06.2013 DE 102013010097
(43) Veröffentlichungstag der Anmeldung: 03.12.2014
(73) Patentinhaber: W.O.M. World of Medicine GmbH, 10587 Berlin (DE)
(72) Erfinder: KÖTH, Yves, 12683 Berlin (DE)
(74) Vertreter: Jungblut & Seuss
(86) Internationale Anmeldenummer: PCT/DE2014/000017
(87) Internationale Veröffentlichungsnummer: WO 2014/111083

(56) Entgegenhaltungen:
- WO-A1-02/32486
- WO-A1-2008/095245
- WO-A1-2009/015410
- WO-A1-2011/078701
- WO-A1-2013/137753
- FR-A1- 2 250 542
- US-A1- 2003 181 857
- US-A1- 2004 254 524

## Beschreibung

Die vorliegende Erfindung betrifft einen Schlauch mit integriertem Heizelement für die Laparoskopie. Mittels eines Befeuchtungsmittels innerhalb des Schlauches wird das im Rahmen der Laparoskopie eingebrachte Gas temperiert und befeuchtet.

### Hintergrund und Stand der Technik

Die Laparoskopie ist ein medizinischer Eingriff bei dem die Bauchhöhle und die darin liegenden Organe visuell überprüft werden können. Hierzu werden üblicherweise kleine Hautschnitte (0,3 - 2 cm) in die Bauchdecke gemacht und durch diese ein Trokar eingebracht, welcher wiederum eine optische Vorrichtung aufnehmen kann. Mit Hilfe eines speziellen Endoskops (Laparoskop) kann der Bauchraum eingesehen werden. Bei der diagnostischen Laparoskopie wird der Bauchraum lediglich visuell inspiziert, im Rahmen eines therapeutischen Vorgriffs können auch operative Eingriffe vorgenommen werden.

Üblicherweise wird zu Beginn der Laparoskopie zunächst der Bauchraum mit Gas befüllt, um ein Pneumoperitoneum zu schaffen. Hierzu sind bereits verschiedene Gase verwendet worden, wie zum Beispiel Luft, Stickstoff oder Kohlendioxid (CO₂). Die Verwendung von Kohlendioxidgas hat sich besonders gut bewährt. Es wurde festgestellt, dass es, insbesondere bei längeren laparoskopischen Eingriffen sinnvoll ist, das eingeführte Gas einerseits zu erwärmen und andererseits zu befeuchten. Die Gaserwärmung dient dazu, den Patienten nicht abzukühlen, sowie ein diffuses Schmerzgefühl des Patienten zu vermeiden, welches wahrscheinlich eine Folge lokaler Abkühlung in Folge des Eintritts von kaltem Gas ist. Die Befeuchtung dient dazu, einem Austrocknen der inneren Bauchoberflächen vorzubeugen, um die dabei entstehende Abkühlung zu vermeiden. Wichtig hierbei ist es, während der Laparoskopie eine relative Gasfeuchtigkeit von über 90% zu erzielen, Dabei ergibt sich bei der Anwendung in der Laparoskopie die Besonderheit, dass die Volumenströme stark schwanken. So kann ein durchschnittlicher Gasfluss von 1-3 l/min. angenommen werden. Sollte jedoch eine größere Leckage auftreten, beispielswiese durch Aktivieren einer Absaugung, werden sofort Gasflussraten > 20 l/min. gefordert, wobei auch diese den geforderten Feuchtigkeitswert von mehr als 90% erreichen sollen.

Hierzu sind im Stand der Technik bereits Anregungen gegeben. So beschreibt beispielsweise die deutsche Patentschrift DE 19510710 eine Vorrichtung, die ein Mittel zur Anpassung der Gasfeuchte vorsieht (beispielsweise einen Schwamm) und weiche optional ein zusätzliches Heizelement enthalten kann.

US 6,068,609 offenbart eine alternative Vorrichtung mit einer Kammer, die einerseits ein schwammartiges Material aufweist, andererseits eine Widerstandsheizung vorsieht. Die Befeuchtungskammer enthält einen Luer-Lock-Anschluss, welcher die Befüllung der Kammer mit Wasser gestattet. Di**e** Kammer der US 6,068,609 wird durch entsprechende Anschlüsse in den Gasstrom der Insufflationseinrichtung eingebracht. Weiteren Stand der Technik bilden die Schriften EP 0827417B1, US 2010/0206308 A1, DE 4331559 A1 und DE 4211986 A1.

US 2003/0181857 A1 offenbart einen Insufflationsschlauch zur Verwendung in der Laparoskopie mit einem Heizelement und mit einem Befeuchtungsmaterial, wobei das Heizelement in unmittelbarer Nähe des Befeuchtungsmaterials positioniert ist.

US 2004/0254524 A1 offenbart einen Insufflationsschlauch zur Verwendung in der Laparoskopie mit einem Heizelement und mit einem Befeuchtungsmaterial, wobei das Heizelement in unmittelbarer Nähe des Befeuchtungsmaterials positioniert ist.

Die im Stand der Technik bekannten Vorrichtungen weisen zunächst technische Nachteile auf.

Zum einen vermindert die patientenseitig angeordnete Kammer die Handhabbarkeit des Insufflationsschlauches während des operativen Eingriffs. Aufgrund der Größe und des Gewichts der Kammer kann diese im nahen OP-Feld den Arzt stören. Weiterhin sind die Befeuchtungsraten des Gases bei verschiedenen Flussraten nicht gleichmäßig hoch zu halten. Insbesondere der kurze Weg des Gases durch die Kammer verhindert bei hohen Flussraten eine optimale Befeuchtung.

Um die Befeuchtungsleistung zu erhöhen wird in den o.g. Lösungen das Gas durch ein Material (beispielsweise einen Schwamm) geleitet. Damit erhöht sich der Gegendruck des Schlauches erheblich und die maximale Flussleistung sinkt. Dies hat erhebliche Nachteile beim Aufrechterhalten des Pneumoperitoneum. Insbesondere wenn hohe Nachfüllraten erforderlich sind (beispielsweise beim Einsatz von Saugpumpen), kann die Insufflationsleistung u.U. nicht mehr ausreichen und der Druck in der Bauchhöhle nicht aufrecht erhalten werden.

Die Einbringung der zusätzlichen Kammer zu Befeuchtung des Gases wirkt sich auch nachteilig auf die Herstellkosten des Insufflationsschlauches aus. Die zusätzlich benötigen Teile erhöhen die Kosten erheblich.

Aufgabe der vorliegenden Erfindung ist es daher, eine verbesserte Vorrichtung zur Erwärmung und Befeuchtung von Insufflationsgasen zur Verfügung zu stellen, welche die genannten Nachteile überwindet.

### Lösung der Aufgabe

Die Lösung der Aufgabe erfolgt durch den Gegenstand der Patentansprüche, d. h. einen Insufflationsschlauch mit integrierter Heiz- und Befeuchtungseinrichtung. Der erfindungsgemäße Schlauch (11) enthält dabei ein Heizelement (12), welches beispielsweise in Form einer Widerstandsheizung das Insufflationsgas (beispielsweise CO₂) erwärmen kann.

Die Heizleistung des Heizelementes (11) muss regelbar sein, da je nach aktueller Befeuchtungsrate sehr unterschiedliche Energiemengen für die Erwärmung des Gases benötigt werden. Der erfindungsgemäße Schlauch (11) kann dazu eine Temperatursonde vorsehen, die vorzugsweise an dem patientenseitigen Ende des Schlauches positioniert ist. Durch die Sonde kann sichergestellt werden, dass die durch den Schlauch strömende Luft ausgangsseitig eine Temperatur von 37°C nicht überschreitet.

Erfindungsgemäß wird zur Erwärmung ein Heizdraht als Heizelement (11) in dem Insufflationsschlauch angebracht. Zur Erhöhung der Leistung kann der Heizdraht innerhalb des Schlauches gewendelt werden. Die damit entstehende Verlängerung des Heizdrahtes führt zur Erhöhung der Oberfläche und damit bei gleichbleibendem Widerstand oder angepasster Spannung zu einer Steigerung der Heizleistung.

Der Insufflationsschlauch (11) kann aus jedem Material bestehen, welches im medizinischen Bereich üblicherweise Verwendung findet, beispielsweise PVC, PUR, TPU oder Silikon. In diesem Bereich übliche Schlauchdurchmesser betragen 6-14 mm.

Der Heizdraht kann aus beliebigen leitenden Materialien, insbesondere Metallen und Metalllegierungen bestehen, z.B. Eisen, Nickel, Chrom oder Kupfer. Der Durchmesser des Drahtes beträgt beispielsweise 0,25 - 1 mm. Bevorzugt wird der Draht in Form einer Wendel mit einem Durchmesser von 3-4 mm verwendet. Der Draht hat bei üblichen Verwendungen eine Länge von 50 cm - 10 m. Bei einer angelegten Spannung von 5-25 V kann so eine Heizleistung von 5-50 W erzielt werden. Vorzugsweise weist der Heizdraht eine elektrische Isolierung auf, um Kurzschlüsse zu vermeiden.

In unmittelbarer Nähe des Heizelementes (12), im Inneren des Schlauches (11), wird darüber hinaus ein Befeuchtungsmaterial (13) positioniert. Es handelt sich dabei um ein poröses Material welches in der Lage ist Flüssigkeit, insbesondere Wasser aufzunehmen. Dieses Befeuchtungsmaterial (13) kann beispielsweise das Heizelement umschließen, so dass das Heizelement (12) im Wesentlichen über seine gesamte Länge direkten Kontakt mit dem Befeuchtungsmaterial (13) hat. Als Befeuchtungsmaterial (13) kann im einfachsten Fall (sterilisierte) Baumwolle verwendet werden, die in der Lage ist eine gewisse Menge Wasser aufzunehmen. Alternativ können folgende Materialien verwendet werden: Schwämme, superabsorbierende Polymere (SAP), Löschpapier, Material aus Phenolharzen. Weitere Materialien sind denkbar.

Die Anordnung des Befeuchtungsmaterials (13) kann über die gesamte Länge des Insufflationsschlauches (11) von 1 - 4 Meter (bevorzugte Länge 2,5 - 3,5 m) erfolgen. Alternativ kann das Material auch über eine kürzere Distanz (beispielsweise nur 40 - 60 cm) im Schlauch (11) angebracht werden. Im letzteren Fall wäre darauf zu achten, dass der Schlauchteil mit Heizungs- (12) und Befeuchtungsvorrichtung (13) vorzugsweise am patientenseitigen Ende des Schlauches positioniert ist. Erfindungsgemäß sollte der Schlauchteil mit Heiz- und Befeuchtungsvorrichtung (12, 13) wenigstens 40 cm Länge und wenigstens 15% der gesamten Schlauchlänge aufweisen.

Je mehr Abstand das Befeuchtungsmaterial (13) zum Schlauch (11) aufweist, umso so weniger Gegendruck erzeugt der Schlauch (11) beim Insufflieren. Der Abstand kann auch stark verringert werden. Damit steigt je nach Art des verwendeten Materials der Gegendruck. Der Fachmann auf dem Gebiet kann in einfacher Weise die einzelnen Parameter variieren um zu einer günstigen Ausführungsform zu gelangen. Aus Sicherheitsgründen wird man den Druck im Schlauch (11) immer begrenzen wollen. Um den gewünschten Gasfluss von bis zu 50 l/min, bei einer gewünschten relativen Gasbefeuchtung von über 90% zu erreichen wird der Fachmann Materialien und Abstände so wählen, dass ein Druck von < 50 mm Hg bevorzugt < 20 mm Hg ausreichend ist.

In einer möglichen Ausführungsform der Erfindung wird der Heizdraht (12) in Form einer Wendel über die gesamte Länge der Wendel mit einem Baumwollgewebe umwickelt.

Die Wasseraufnahmekapazität des porösen Materials ist naturgemäß abhängig von dem jeweiligen Material. Für eine normale Operation werden ca. 200 Liter Gas verbraucht. Um dieses auf annähernd 100% relative Feuchtigkeit zu befeuchten werden -10 ml Flüssigkeit benötigt. Es ist vorteilhaft, wenn die Menge des verwendeten Befeuchtungsmaterials (13) diese Flüssigkeitsmenge aufnehmen kann.

Je nach geplanter Dauer des laparoskopischen Eingriffes und dem Gasfluss kann es ausreichend sein, das poröse Material einmal vor der Laparoskopie zu befeuchten. Insbesondere bei länger andauernden Operationen kann eine weitere Befeuchtung nötig sein. Hierzu kann der Schlauch (11) eine optionale Zuleitung vorsehen, welche die weitere Einbringung von Wasser ermöglicht. Für den Fachmann auf dem Gebiet versteht sich von selbst, dass sowohl das vor Beginn der Operation, als auch ggf. während der Operation gegebenes Wasser steril sein muss.

In einer alternativen Ausführungsform der Erfindung kann das Heizelement (12) auch so ausgestaltet sein, dass eine Widerstandsänderung des Heizelementes (12) mit der Erwärmung auftritt. Durch Messung des Widerstandes kann in diesem Fall die Temperatur des Heizelementes bestimmt werden. Hierdurch kann die Einbringung einer zusätzlichen Temperatursonde ggf. vermieden werden.

Der erfindungsgemäße Insufflationsschlauch (11) weist den Vorteil auf, dass bis auf das Befeuchtungsmaterial (13) keine zusätzlichen Bauelemente und hierzu passende Anschlüsse nötig sind, so dass der Schlauch in einem Stück gefertigt werden kann. Damit kann der Schlauch preiswerter hergestellt werden, als die Lösungen, die im Stand der Technik genannt sind.

Weiterhin ergibt sich im Vergleich zu einem normalen beheizten Insufflationsschlauch (11) kein Unterschied in der Handhabbarkeit für den Arzt während der operativen Eingriffs.

Durch den geringen Gegendruck, den der Schlauch (11) aufbaut, können hohe Flussraten von 40-50 l/min und mehr erzeugt werden.

Durch die längere Verweilzeit des Gases an der Befeuchtungseinrichtung kann auch bei den hohen Flussraten eine hohe Befeuchtungsrate (über 90% rel. Gasfeuchtigkeit) erreicht werden.

Der Schlauch kann aus den üblichen, im medizinischen Bereich verwendeten Kunststoffen hergestellt sein, wie beispielsweise Silikon, TPU, PUR oder PVC.

In der erfindungsgemäßen Ausführungsform enthält der Insufflationsschlauch (11) in seinem Inneren einen weiteren Schlauch (14), durch den das Insufflationsgas in den Insufflationsschauch (11) eingetragen wird. Dieser innere Schlauch (14) ist gasdurchlässig, so dass der Gasdurchtritt gewährleistet ist. Dies erfolgt erfindungsgemäß dadurch, dass der Innenschlauch (14) auf seiner Mantelfläche eine Vielzahl von Öffnungen (15) enthält, so dass das Insufflationsgas in radialer Richtung ausströmen kann. Hierzu kann zum Beispiel ein sogenannter Geflechtschlauch Anwendung finden, der in verschiedensten Ausführungsformen erhältlich ist. Übliche Materialien für diesen inneren Schlauch (14) sind die oben genannten, vorzugsweise Silikon, TPU, PUR oder PVC, wobei auch ein Drahtgeflecht denkbar wäre. Der Innenschlauch (14) mit den radialen Austrittsöffnungen (15) ist erfindungsgemäß ummantelt mit dem Befeuchtungsmaterial (13) und dem Heizelement (12). Dies kann beispielsweise so erfolgen, dass der Innenschlauch (14) zunächst mit einem gewendelten Nickelchromdraht ummantelt ist und dann das Befeuchtungsmaterial (13) wiederum das Heizelement (12) ummantelt. In einer alternativen Ausführungsform kann auch zunächst eine Schicht Befeuchtungsmaterial (13) als Mantel des Innenschlauches (14) dienen. Dieses Befeuchtungsmaterial (13) kann dann von dem Heizelement (12) wendelförmig ummantelt sein. In dieser Ausführungsform bildet eine zweite Schicht Befeuchtungsmaterial (13) einen weiteren Mantel. Allen diesen Ausführungsformen ist gemein, dass über die gesamte Länge des Innenschlauches (14) das Insufflationsgas vor allem in radialer Richtung ausströmt und hierbei sowohl erhitzt, als auch befeuchtet wird. Aufgrund der besseren Feuchtigkeitsaufnahme eines wärmeren Gases ist zu bevorzugen, dass zunächst die Heizung, dann die Befeuchtung stattfindet.

### Beispiele

Die vorliegende Erfindung wird durch das folgende Beispiel näher erläutert, ohne dass dieses einschränkend sein soll. Der Fachmann auf dem Gebiet ist ohne weiteres in der Lage weitere vorteilhafte Ausführungsformen zu bilden, ohne erfinderisch tätig werden zu müssen.

### Beispiel 1 (nicht erfindungsgemäß, nur zur Erläuterung»

In einem Schlauch, bestehend aus PVC, welcher eine Länge von 3m aufweist, wird über eine Strecke von 90 cm ein gewendeltes Heizelement positioniert. Das Heizelement besteht aus Nickelchrom. Das Heizelement ist über eine elektrische Zuund Ableitung mit Strom versorgt. Dabei wird mittels einer Spannung von 24 V eine elektrische Heizleitung von ∼30 Watt erzielt.

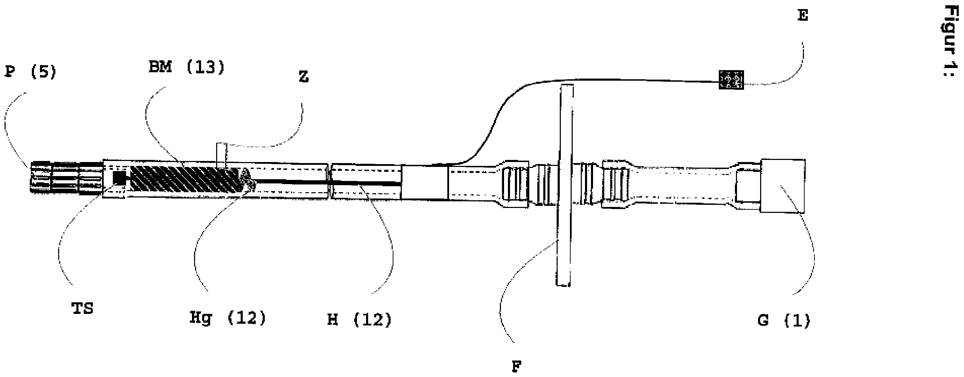

Das Heizelement ist mit steriler Mullbinde als Befeuchtungsmaterial umwickelt, so dass das Heizelement im Wesentlichen überall unmittelbaren Kontakt mit dem Befeuchtungsmaterial hat. Hierbei handelt es sich um folgendes Material Baumwolle 68%, Polyamid 24%, Elastan 8%. Zusätzlich ist eine Temperatursonde am Ausgang des Schlauches angebracht (siehe Figur 1).

Vor Beginn der Laparoskopie wird das poröse Material mit ∼10 ml sterilem Wasser befeuchtet Die Zufuhr des Wassers erfolgt über den zusätzlichen Zugang/über das patientenseitige Ende des Schlauches. Der Schlauch wird an seinem patientenseitigen Ende mit einer Veress-Nadel versehen, die in den Bauchraum des Patienten eingeführt wird. Vor der Einführung wird durch die im Schlauch vorhandene Temperatursonde sichergestellt, dass die Gastemperatur ausgangsseitig nicht höher ist als 37°C. Über den Schlauch kann ein Gasstrom von bis zu 50l/min in den Patienten zugeführt, wobei dieser bei Verwendung von Instrumenten, beispielsweise einer Veresskanüle, deutlich sinken kann.

Der laparoskopische Eingriff unter Verwendung des erfindungsgemäßen Schlauches kann bis zu 60 min. dauern, wobei jeweils nach 200 Litern Gasverbrauch ca. 10 ml Wasser über die Zuleitung nachgefüllt werden.

### Beispiel 2 (erfindungsgemäß)

Ein Geflechtschlauch (14) mit einer Länge von 50 cm aus dem Material PET (Durchmesser 3 mm), der über die gesamte Länge eine Vielzahl von Öffnungen (15, < 0,5 mm) aufweist, wird mit einem Nickelchromdraht (12) ummantelt. Über den Nickelchromdraht (12) wird eine Schicht eines sterilen Baumwollgewebes (13) aufgetragen. Der in dieser Weise entstandene ummantelte Schlauch wird in einen PVC-Schlauch (11) von 3 m Länge eingeführt (Durchmesser 10 mm). Das Heizelement (12) wird über eine elektrische Zu- und Ableitung mit Strom versorgt. Dabei wird mittels einer Spannung von 24 Volt eine elektrische Heizleistung von ca. 50 Watt erzielt. Das oben genannte Befeuchtungsmaterial (13) enthält neben Baumwolle auch Polyamid und Elastan (s. Beispiel 1). Weiterhin ist eine Temperatursonde am Ausgang des Schlauches angebracht.
Die Gaszufuhr erfolgt ausschließlich über den Innenschlauch (14).

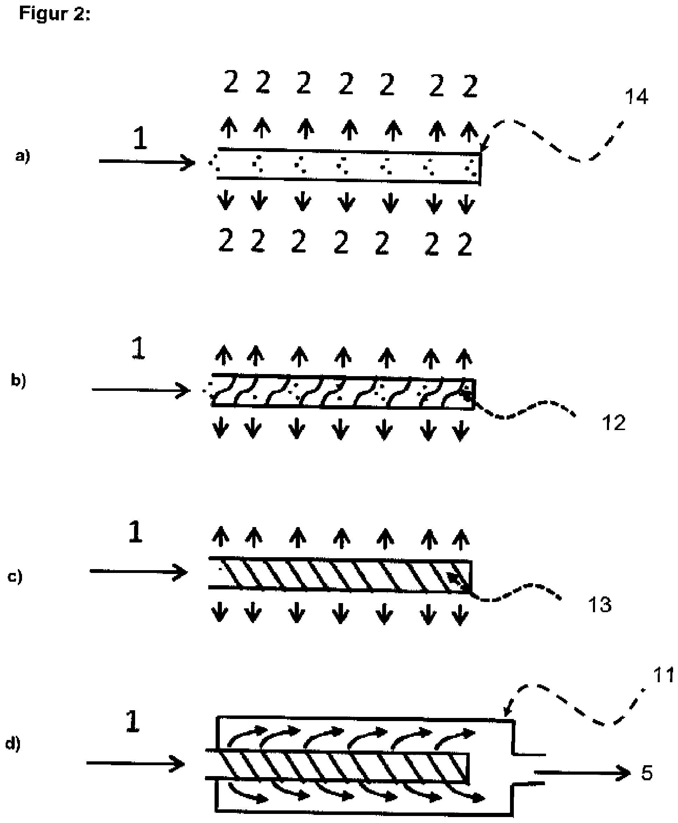

Ein derartiger Insufflationsschlauch (11) gemäß Beispiel 2 ist in der Figur 2 abgebildet. Figur 2a zeigt einen Schlauch (14), der auf der Mantelfläche eine Vielzahl von Öffnungen (15) aufweist. Die Gaszufuhr (1) ist im Bild links dargestellt. Am gegenüberliegenden Ende des Schlauches (17) ist dieser verschlossen, so dass der Gasaustritt über die Vielzahl von Öffnungen (15) in radialer Richtung erfolgt (2). Figur 2b zeigt die wendelförmige Ummantelung des Schlauches mit dem Heizdraht (12). Figur 2c zeigt die weitere Ummantelung mit dem Befeuchtungsmaterial (13). Das in Figur 2c dargestellte Konstrukt wird in einen größeren Schlauch eingeführt und mit diesem verbunden, so dass der Gaseinlass (1) lediglich über den Innenschlauch (14) erfolgt. Das einströmende Gas wird über den Heizdraht (12) erwärmt und mittels des Befeuchtungsmaterials (13) befeuchtet und strömt dann aus dem Ende des Außenschlauches (11).

Vor Beginn der Laparoskopie wird das Befeuchtungsmaterial (13) mit ca. 10 ml sterilem Wasser befeuchtet Die Zufuhr des Wassers erfolgt entweder über einen zusätzlichen Zugang oder über das patientenseitige Ende des Schlauches (5). Der Schlauch wird an seinem patientenseitigen Ende mit einer Veress-Nadel versehen, die in den Bauchraum des Patienten eingeführt wird. Vor der Einführung wird durch die im Schlauch vorhandene Temperatursonde (TS) sichergestellt, dass die Gastemperatur ausgangsseitig nicht höher ist als 37°C. Über den Schlauch (11) wird ein Gasstrom von bis zu 40 l/min in den Patienten zugeführt, wobei der Druck im Innenschlauch (14) 30 mmHg nicht übersteigt. Der laparoskopische Eingriff unter Verwendung des erfindungsgemäßen Schlauches kann bis zu 60 min dauern, wobei jeweils nach 200 l Gasverbrauch ca. 10 ml Wasser über die Zuleitung nachgefüllt werden.

### Bezugszeichenliste zu Figur 1:

- P: zum Patienten
- BM: Befeuchtungsmaterial (13)
- Z: Zuleitung
- E: Elektrischer Anschluß
- TS: Temperatursonde
- Hg: Heizdraht (12), gewendelt
- H: Heizdraht (12)
- F: Filter
- G: zum Gerät

### Bezugszeichenliste zu Figur 2a:

- 1: Gasstrom in den Innenschlauch (14) mit verschlossenenem Ende (16) und Vielzahl von Öffnungen (15) auf der Mantelfläche (gepunktet dargestellt)
- 2: Gasaustritt aus den Öffnungen des Innenschlauches (14) in radialer Richtung

### Bezugszeichenliste zu Figur 2b:

- 12: Ummantelung des Innenschlauches (14) aus Figur 2a mit Heizdraht (12)

### Bezugszeichenliste zu Figur 2c:

- 13: Weitere Ummantelung des Innenschlauches (14) mit Heizdraht (12) aus Figur 2b mit Befeuchtungsmaterial (13)

### Bezugszeichenliste zu Figur 2d:

- 11: Insufflationsschlauch, mit Konstrukt aus Fig 2c im Schlauchinneren
- 5: Schlauchausgang zum Patienten

## Patentansprüche

1. Insufflationsschlauch (11) zur Verwendung in der Laparoskopie mit einem Heizelement (12) und mit einem Befeuchtungsmaterial (13), wobei sich im Schlauchinneren ein Innenschlauch (14) befindet, wobei der Innenschlauch (14) eine Vielzahl von öffnungen (15) auf der Mantelfläche aufweist,
wobei der Innenschlauch (14) mit dem Heizelement (12) und dem Befeuchtungsmaterial (13) ummantelt ist, so dass sich der Innenschlauch (14) mit dem Heizelement (12) und dem Befeuchtungsmaterial (13) im Inneren des Insufflationsschlauches befinden und wobei das Heizelement (12) in unmittelbarer Nähe des Befeuchtungsmaterials (13) positioniert ist,
wobei der Innenschlauch (14) an einem Ende (16) einen Gaseinlass aufweist, durch den das Insufflationsgas in den Insufflationsschlauch eingetragen wird und wobei der Innenschlauch (14) an seinem anderen Ende (17) verschlossen ist,
wobei die Länge des Insufflationsschlauches (11) 1-4 m beträgt,
wobei der Schlauchteil mit Heizelement (12) und Befeuchtungsmaterial (13) wenigstens 40 cm Länge und wenigstens 15% der gesamten Schlauchlänge aufweisen.

2. Insufflationsschlauch (11) gemäß Anspruch 1, **gekennzeichnet durch** eine Temperatursonde (18), mittels derer die Leistung des Heizelementes (12) geregelt werden kann.

3. Insufflationsschlauch (11) gemäß Anspruch 1, wobei das Heizelement (12) einen temperaturabhängigen Widerstand aufweist, der eine Temperaturmessung während der Insufflation erlaubt.

4. Insufflationsschlauch (11) gemäß Anspruch 2 oder 3, **gekennzeichnet durch** einen separaten Zugang zum prä- und intraoperativen Befeuchten des Befeuchtungsmaterials (13).

5. Insufflationsschlauch (11) gemäß mindestens einem der Ansprüche 1-4, wobei das Heizelement (12) aus einer Drahtwendel besteht.

6. Insufflationsschlauch (11) gemäß mindestens einem der Ansprüche 1-5, wobei das Befeuchtungsmaterial (13) aus einem sterilisierten Baumwollgewebe besteht.

7. Insufflationsschlauch (11) gemäß mindestens einem der Ansprüche 1-6, wobei der Insufflationsschlauch (11) aus PVC, PUR, TPU oder Silikon besteht.

8. Insufflationsschlauch (11) gemäß mindestens einem der Ansprüche 1-7, wobei der Innenschlauch (14) ein Geflechtsschlauch aus Polyethylenterephtalat, PET, ist.

## Claims

1. An insufflation hose (11) for use in laparoscopy, comprising a heating element (12) and a wetting material (13), in the interior of the hose an inner hose (14) being arranged, the inner hose (14) having a multitude of openings (15) on its sheath surface,
wherein the inner hose (14) is sheathed with the heating element (12) and the wetting material (13) such that the inner hose (14) with the heating element (12) and the wetting material (13) is in the interior of the insufflation hose, and wherein the heating element (12) is positioned in immediate vicinity of the wetting material (13),
wherein the inner hose (14) comprises at one end (16) thereof a gas inlet, through which the insufflation gas is supplied to the insufflation hose, and wherein the inner hose (14) at the other end (17) thereof is closed,
wherein the length of the insufflation hose (11) is 1 to 4 m,
wherein the hose portion with heating element (12) and wetting material (13) has a length of at least 40 cm and at least 15 % of the full hose length.

2. The insufflation hose (11) according to claim 1, **characterized by** a temperature probe (18), by means of which the power of the heating element (12) can be controlled.

3. The insufflation hose (11) according to claim 1, wherein the heating element (12) includes a temperature-dependent resistor that allows for a temperature measurement during insufflation.

4. The insufflation hose (11) according to claim 2 or 3, **characterized by** a separate access for preoperative and intraoperative wetting of the wetting material (13).

5. The insufflation hose (11) according to at least one of claims 1 to 4, wherein the heating element (12) consists of a wire helix.

6. The insufflation hose (11) according to at least one of claims 1 to 5, wherein the wetting material (13) consists of a sterilized cotton fabric.

7. The insufflation hose (11) according to at least one of claims 1 to 6, wherein the insufflation hose (11) consists of PVC, PUR, TPU, or silicone.

8. The insufflation hose (11) according to at least one of claims 1 to 7, wherein the inner hose (14) is a braid hose made of polyethylene terephthalate, PET.

## Revendications

1. Tuyau d'insufflation (11) pour une utilisation dans la laparoscopie, comprenant un élément de chauffage (12) et un matériau de mouillage (13), dans l'intérieur du tuyau un tuyau intérieur (14) étant disposé, le tuyau intérieur (14) comportant une pluralité d'ouvertures (15) sur la surface de sa gaine,
dans lequel le tuyau intérieur (14) est gainé avec l'élément de chauffage (12) et le matériau de mouillage (13) de façon que le tuyau intérieur (14) avec l'élément de chauffage (12) et le matériau de mouillage (13) soit à l'intérieur du tuyau d'insufflation, et dans lequel l'élément de chauffage (12) est positionné à proximité immédiate du matériau de mouillage (13),
dans lequel le tuyau intérieur (14) comprend à une extrémité (16) de celui-ci une entrée de gaz, à travers laquelle le gaz d'insufflation est fourni au tuyau d'insufflation, et dans lequel le tuyau intérieur (14) est fermé à l'autre extrémité (17) de celui-ci,
dans lequel la longueur du tuyau d'insufflation (11) est de 1 à 4 m,
dans lequel la partie du tuyau avec l'élément de chauffage (12) et le matériau de mouillage (13) a une longueur d'au moins 40 cm et d'au moins 15 % de la longueur complète du tuyau.

2. Tuyau d'insufflation (11) selon la revendication 1, **caractérisé par** une sonde de température (18), au moyen de laquelle la puissance de l'élément de chauffage (12) peut être réglée.

3. Tuyau d'insufflation (11) selon la revendication 1, dans lequel l'élément de chauffage (12) comporte une thermistance permettant le mesurage de la température pendant l'insufflation.

4. Tuyau d'insufflation (11) selon la revendication 2 ou 3, **caractérisé par** un accès séparé pour le mouillage pré-opératoire et intra-opératoire du matériau de mouillage (13).

5. Tuyau d'insufflation (11) selon au moins une des revendications 1 à 4, dans lequel l'élément de chauffage (12) consiste en une hélice de fil.

6. Tuyau d'insufflation (11) selon au moins une des revendications 1 à 5, dans lequel le matériau de mouillage (13) consiste en un tissu de coton stérilisé.

7. Tuyau d'insufflation (11) selon au moins une des revendications 1 à 6, dans lequel le tuyau d'insufflation (11) consiste en PVC, PUR, TPU, ou silicone.

8. Tuyau d'insufflation (11) selon au moins une des revendications 1 à 7, dans lequel le tuyau intérieur (14) est un tuyau en tressage en polyéthylène téréphthalate, PET.
